# EUROPEAN PATENT SPECIFICATION

(11) **EP 3 711 066 B1**
(45) Date of publication and mention of the grant of the patent: **04.11.2026**
(21) Application number: 18800651.4
(22) Date of filing: 15.11.2018
(51) Int. Cl.: G16H 40/67, G06F 11/07, G06F 11/16

(54) **METHOD FOR CONTROLLING OPERATION OF A MEDICAL DEVICE IN A MEDICAL SYSTEM AND MEDICAL SYSTEM**
VERFAHREN ZUR STEUERUNG DES BETRIEBS EINER MEDIZINISCHEN VORRICHTUNG IN EINEM MEDIZINISCHEN SYSTEM SOWIE MEDIZINISCHES SYSTEM
PROCÉDÉ POUR COMMANDER LE FONCTIONNEMENT D'UN DISPOSITIF MÉDICAL DANS UN SYSTÈME MÉDICAL ET SYSTÈME MÉDICAL

(30) Priority: 17.11.2017 EP 17202302
(43) Date of publication of application: 23.09.2020
(73) Proprietor: Roche Diabetes Care GmbH, 68305 Mannheim (DE); F. Hoffmann-La Roche AG, 4070 Basel (CH)
(72) Inventor: FRIEDLI, Kurt, 68305 Mannheim (DE); MUELLER, Michael, 68305 Mannheim (DE); DANCKWARDT, Nils, 68305 Mannheim (DE)
(74) Representative: Bittner, Thomas L.
(86) International application number: PCT/EP2018/081308
(87) International publication number: WO 2019/096876

(56) References cited:
- US-A- 5 915 082
- US-A1- 2008 256 076
- US-A1- 2012 066 551
- US-A1- 2016 034 658
- CHRISTOPHER PRESCHERN ET AL: "Building a safety architecture pattern system", PATTERN LANGUAGES OF PROGRAM, ACM, 2 PENN PLAZA, SUITE 701 NEW YORK NY 10121-0701 USA, 10 July 2015 (2015-07-10), pages 1 - 55, XP058070859, ISBN: 978-1-4503-3465-5, DOI: 10.1145/2739011.2739028

## Description

The present disclosure refers to a method for controlling operation of a medical device in a medical system and a medical system.

### Background

Medical devices in which a malfunction can lead to a hazard to a user of the medical device must adhere to functional safety principles to comply with normative requirements. Non-medical devices do not usually adhere to these principles. If a medical device, for example an insulin infusion pump, is to be controlled by a non-medical device such as a mobile phone, functional safety of the medical device has to be ensured.

Document US 2015 / 0182694 A1 discloses a safety processor that acts as an intermediary device between a remote control device, such as a smartphone, and a medical device to review transmissions from the smartphone prior to the transmissions being delivered to the medical device. The safety processor can determine whether the smartphone is compatible with the medical device by checking the type and version of the smartphone as well as the versions of the operating software and / or firmware resident on the phone. The safety processor can also check whether the operating command entered into the smartphone is within acceptable parameters.

Systems and methods for remote control and / or wireless (re-)programming of drug pump devices are described in document US 2011 / 0275410 A1. In various embodiments, a smartphone or other wireless handheld device is used to send control commands and / or drug-delivery protocols to a wireless receiver of the drug pump device, e.g., via a Wi-Fi, Blue-tooth, or near-field communication link. The smartphone may be equipped with a dongle to provide security and / or flexibility in the choice of the data-transfer protocol. Further, the smartphone may store a special software application that enables interaction with the user, and which may have security features that prevent unauthorized operation.

For a safe remote programming of a pump infusion system, document US 2011 / 0275410 A1 discloses assigning a predetermined program, comparing a protocol to be programmed with a protocol coming from the pump, eliminating possible errors of non-medically certified intervening devices and means, and executing the programming.

Document US 6,128,774 describes a computer-implemented method of verifying that untrusted software supplied by a code producer is safe to execute by a code consumer. The method includes the step of defining a safety policy that specifies safe operating conditions of the untrusted software on the code consumer. The method also includes the steps of generating a safety predicate for the untrusted software that determines if execution by the code consumer of the untrusted software will violate said safety policy and generating a safety proof that proves that said safety predicate is valid. The method further includes the step of validating the untrusted software for execution based on said safety proof and said safety predicate.

According to document US 2011 / 0126188 A1, methods and articles of manufacture for hosting a safety critical application (SCA) on an uncontrolled data processing device (UDPD) are provided. Various combinations of installation, functional, host integrity, coexistence, interoperability, power management, and environment checks are performed at various times to determine if the safety critical application operates properly on the device. The operation of the SCA on the UDPD may be controlled accordingly.

Methods and systems for validating safety critical applications (SCAs) on uncontrolled data processing devices (UDPDs) are disclosed also in document US 2016 / 034658 A1. Various combinations of checks including validation of safety critical features, validation of SCA-UDPD compatibility, and resource management are executed at various times to ensure the SCA operates properly on the device. The operation of the SCA on the UDPD may be controlled accordingly.

According to document US 2012 / 066551 A1 safe operation in a processor may be verified by making use of an execution trace module that is normally only used for testing and software development. During operation of the processor in the field, a sequence of instructions may be executed the processor. A portion of the execution is traced to form a sequence of trace data. The sequence of trace data is compressed to form a checksum. The checksum is compared to a reference checksum, and an execution error is indicated when the checksum does not match the reference checksum.

A method and system of controlling access to a system in a medical environment is disclosed in document US 2008 / 256076 A1. The method includes calculating a signature value for at least one file usable with the medical system, transferring the calculated signature value to a signature file, and providing at least one signature value in the signature file and at least one associated file to a file system configured to be received by the medical system. At least one signature value and at least one associated file are inspected by the medical system to verify the associated file is a known medical software application asset. The medical system comprises an input/output data port configured to receive the external memory storage device, and an operating system capable of reading medical system data from and writing medical system data to the memory storage device.

A lockstep processor system is known from document US 5,915,082, which adds error detection, isolation, and recovery logic to one or more lockstep processor system functions; namely, control outputs, processor inputs, I/O busses, memory address busses, and memory data busses.

### Summary

It is an object of the present disclosure to provide a method for controlling operation of a medical device in a medical system that provides enhanced safety and security with regard to operation of and communication in the medical system. Further, a medical system shall be provided.

For solving the problem, a method for controlling operation of a medical device in a medical system according to the independent claim 1 is proposed. Further, a medical system according to claim 14 is provided. Alternative embodiments are the subject of dependent claims.

According to an aspect, a method for controlling operation of a medical device in a medical system is provided. The medical system is having a medical device, a communication device provided with a medical device application running on the communication device and adapted for remote operation control of the medical device, and a safety device adapted for data communication with the communication device. The method, in the medical system, comprises: providing input data for a calculation process; processing the input data by the calculation process in a first calculation process in the communication device, thereby, providing first calculation result data; processing the input data by the calculation process in a second calculation process executed separately of the first calculation process, thereby, providing second calculation result data; comparing the first and second calculation result data; if the first and second calculation result data are found equal, allowing remote operation control of the medical device by the medical device application running on the communication device; and if the first and second calculation result data are found not equal, preventing the medical device application running on the communication device from remote operation control of the medical device.

According to another aspect, a medical system is provided. The medical system comprises a medical device, a communication device provided with a medical device application running on the communication device and adapted for remote operation control of the medical device, and a safety device adapted for data communication with the communication device. The system is adapted to provide input data for a calculation process; process the input data by the calculation process in a first calculation process in the communication device, thereby, providing first calculation result data; process the input data by the calculation process in a second calculation process executed separately of the first calculation process, thereby, providing second calculation result data; compare the first and second calculation result data; if the first and second calculation result data are found equal, allow remote operation control of the medical device by the medical device application running on the communication device; and if the first and second calculation result data are found not equal, prevent the medical device application running on the communication device from remote operation control of the medical device.

The first calculation result data and the second calculation result data may also be referred to as the first result data and the second result data, respectively. The safety device may also be referred to as a trusted device.

In general, operational safety of a device may be increased by implementing a diverse redundant architecture. Diverse redundancy is known in the art as such and referenced, for example, in the IEC 61508 and IEC 61511 standards. Diverse redundancy refers to the same task being performed more than once, referred to as redundancy, in separate and different manners, for example using different methodology and / or components, referred to as diversity. One diverse set of means for performing one such task may be referred to as a channel which is separate from a different channel for performing the same task. Separate channels for performing tasks are known in the art as such.

The method comprises providing the input data for the calculation process in both the communication device and the safety device and processing the input data by the calculation process in the second calculation process in the safety device. The first calculation process and the second calculation process are performed in two separate devices. If the first and the second calculation result data are found equal, the two separate devices may therefore be found to have produced the same calculation result after the same input data was provided to the two separate devices. If the first and the second calculation result data are found not equal, it may be assumed that at least one of the first calculation process in the communication device and the second calculation process in the safety device has not been performed correctly.

The method may further comprise receiving the input data in a first device of the communication device and the safety device, encrypting the input data in the first device, transmitting the encrypted input data from the first device to a second device of the communication device and the safety device, and decrypting the encrypted input data in the second device. For example, the input data may be received and encrypted in the communication device. The encrypted input data may then be transmitted to the safety device and be decrypted in the safety device.

A software application for implementing the calculation process is received in a first device of the communication device and the safety device and may be transmitted from the first device to a second device of the communication device and the safety device. The software application may be stored in at least one of a memory and a storage device of the first device and / or the second device. Additionally, the software application may be encrypted in the first device. The encrypted software application may then be transmitted from the first device to the second device and be decrypted in the second device.

The method may comprise providing the input data for the calculation process in the communication device and processing the input data by the calculation process in the second calculation process in the communication device. The first calculation process may be performed in a first channel of the communication device and the second calculation process may be performed in a second channel of the communication device independent of the first channel. If the first and the second calculation result data are found not equal, it may be assumed that at least one of the first calculation process in the first channel of the communication device and the second calculation process in the second channel of the communication device has not been performed correctly.

The method may comprise providing the first and second calculation result data in the safety device and comparing the first and second calculation result data in the safety device. Providing the first and second calculation result data in the safety device may comprise transmitting the first and / or the second calculation result data from the communication device to the safety device.

The method may further comprise providing compare result data in the safety device, the compare result data indicative of the result of comparing the first and second calculation result data, and transmitting the compare result data from the safety device to the communication device. The compare result data may be encrypted in the safety device. The encrypted compare result data may be transmitted from the safety device to the communication device and be decrypted in the communication device. Compare result data may be any data indicative of the result of comparing the first and second calculation result data. For example, compare result data may consist of a single bit transmitted in a data packet, the single bit indicating whether the first and second calculation result data are found equal or not equal. Alternatively, compare result data may comprise additional information, for example the first and second calculation result data. Compare result data may be indicative of a permission for or a denial of remote operation control of the medical device by the medical device application running on the communication device.

In embodiments in which the first calculation process is performed in the communication device, the second calculation process is performed in the safety device, and comparing the first and second calculation result data is performed in the safety device, the method may comprise encrypting the first result data in the communication device, transmitting the encrypted first result data from the communication device to the safety device, and decrypting the encrypted first result data in the safety device.

In embodiments in which the first calculation process and the second calculation process are performed in the communication device and compare result data is provided in the safety device and transmitted from the safety device to the communication device, the method may comprise encrypting the first and second result data in the communication device, transmitting the encrypted first and second result data from the communication device to the safety device, and decrypting the encrypted first and second result data in the safety device.

The first and second calculation result data may be compared in the communication device. Comparing the first and second calculation result data in the communication device may be in addition or as an alternative to comparing the first and second calculation result data in the safety device.

In embodiments in which the first calculation process is performed in the communication device, the second calculation process is performed in the safety device, and comparing the first and second calculation result data is performed in the communication device, the method may comprise encrypting the second result data in the safety device, transmitting the encrypted second result data from the safety device to the communication device, and decrypting the encrypted second result data in the communication device.

The safety device may be selected from the following group: memory card such as SD card; NFC beacon; remote server device; further medical device different from the medical device. The safety device may be a medical device or a non-medical device. The safety device may be provided with a diverse redundant architecture.

For example, the safety device may be a medical device SD card provided with additional functionality in comparison to a standard SD card, which is known in the art as such. The medical device SD card may be connected to the communication device, for example a mobile phone, like a standard SD card and provide the functionality of a standard SD card, such as file services. Additionally, a safe medical comparison / calculation application, providing additional functionality referred to above with reference to the safety device, may be running on an internal microcontroller of the medical device SD card. The medical device SD card may be configured to perform any or all tasks referred to above with regard to the safety device in a diverse redundant manner.

Alternatively, the safety device may be any other device that may be physically connected to the communication device, for example a USB dongle. The USB dongle or other device that may be physically connected to the communication device may provide the functionality described above with regard to the medical SD card.

The safety device may be a device capable of connecting wirelessly to the communication device via near field communication (NFC). NFC is known as such. The safety device may be an NFC beacon that may be attached directly to the communication device. For example, the safety device may be an NFC sticker or a slim NFC-capable device that is small enough to not hinder normal use of the communication device when attached to the communication device.

The method may comprise providing the calculation process and the comparing of the first and second calculation result data as safety elements of a single fault safety architecture. In a single fault safety architecture, an unwanted result, such as a hazard to a user, does not occur even if one (single) fault occurs within the architecture. For example, a hazard to a user of the medical device may be prevented by preventing the medical device application running on the communication device from remote operation control of the medical device if the first and second calculation result data are found not equal when one of the first and second calculation result data is incorrect due to a fault occurring.

The method may comprise storing, in a memory device provided in at least one of the communication device and the safety device, at least one of the following: the input data; the encrypted input data; the first result data; the encrypted first result data; the second result data; and the encrypted second result data.

For example, the encrypted input data, the encrypted first result data and the encrypted second result data may be stored in a memory device of the safety device. The encrypted input data, the encrypted first result data and the encrypted second result data may then be decrypted and the input data, the first result data and the second result data may be stored in the memory device of the safety device. The input data, the first result data and / or the second result data may then be used for comparing the first and second result data. In the same manner, additionally or as an alternative, data may be stored in a memory device of the safety device and used for comparing the first and second result data.

Input reference data may be provided in at least one of the safety device and the communication device. The input reference data may be compared, in at least one of the safety device and the communication device, to the input data to determine whether the input data is valid input data. If the input data is found valid, remote operation control of the medical device by the medical device application running on the communication device may be allowed. If the input data is found not valid, the medical device application running on the communication device may be prevented from remote operation control of the medical device. The comparing the input data to the reference input data may be performed before or after the comparing the first and second calculation result data or, at least partially, at the same time.

The communication device on which the medical device application is running may be any communication device, for example a mobile communication device, such as a mobile phone, a smart watch, a (mobile) computer, or a portable display device. The communication device may be provided with communication functionality. The communication device may be connected, wirelessly or via a wire, to the medical device and / or the safety device.

The medical device may be an insulin pump. Alternatively, the medical device may be a pen device for administering insulin to a patient. As a further alternative, the medical device may be a glucose monitoring device which may be a glucose monitoring device adapted for continuous glucose monitoring or non-continuous glucose monitoring.

A glucose monitoring device adapted for continuous blood glucose monitoring may be provided with a sensor that is a fully or partially implanted sensor for continuous glucose monitoring (CGM). In general, in the context of CGM, an analyte value or level indicative of a glucose value or level in the blood may be determined. The analyte value may be measured in an interstitial fluid. The measurement may be performed subcutaneously or in vivo. CGM may be implemented as a nearly real-time or quasi-continuous monitoring procedure frequently or automatically providing / updating analyte values without user interaction. In an alternative embodiment, analyte may be measured with a biosensor in a contact lens through the eye fluid or with a biosensor on the skin via transdermal measurement in sudor. A CGM sensor may stay in place for several days to weeks and then must be replaced.

The trusted device may be a device that is known by a provider of the medical device application to work safely. For example, the trusted device may have been confirmed by the provider of the medical device application to perform operations necessary for remote control operation of the medical device correctly under all anticipated operation conditions.

With regard to the medical system, the alternative embodiments described above may apply *mutatis mutandis.*

In the medical system, the safety device may be an integrated device of the medical device. For example, the safety device may be a memory device, such as an SD card, provided in the medical device. Alternatively, the safety device may comprise at least one of hardware components and software components provided in the medical device and separate from other components of the medical device.

Again, in the method and in the medical system as described above, the communication device may be a hardware device suitable for running a medical device application thereon and separate from the medical device for remote controlling the medical device. Vice versa, the medical device may be a hardware device separate from the communication device. The communication device and the medical device may be configured to communicate with each other, in particular for remote controlling the medical device.

### Description of embodiments

Following, further embodiments are described with reference to figures. In the figures, show:
- Fig. 1: a schematic representation of a medical system;
- Fig. 2: a schematic representation of a further medical system;
- Fig. 3: a schematic representation of a medical device;
- Fig. 4: a flow diagram for steps of a method for controlling operation of a medical device in a medical system;
- Fig. 5: a flow diagram for steps of a further method for controlling operation of a medical device in a medical system;
- Fig. 6: a flow diagram for steps of a further method for controlling operation of a medical device in a medical system;
- Fig. 7: a flow diagram for steps of a further method for controlling operation of a medical device in a medical system; and
- Fig. 8: a flow diagram for steps of a further method for controlling operation of a medical device in a medical system.

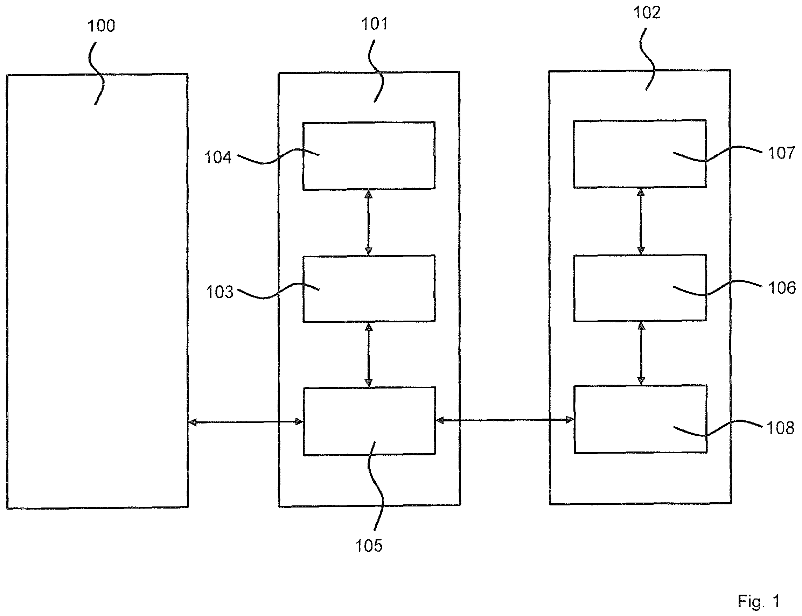

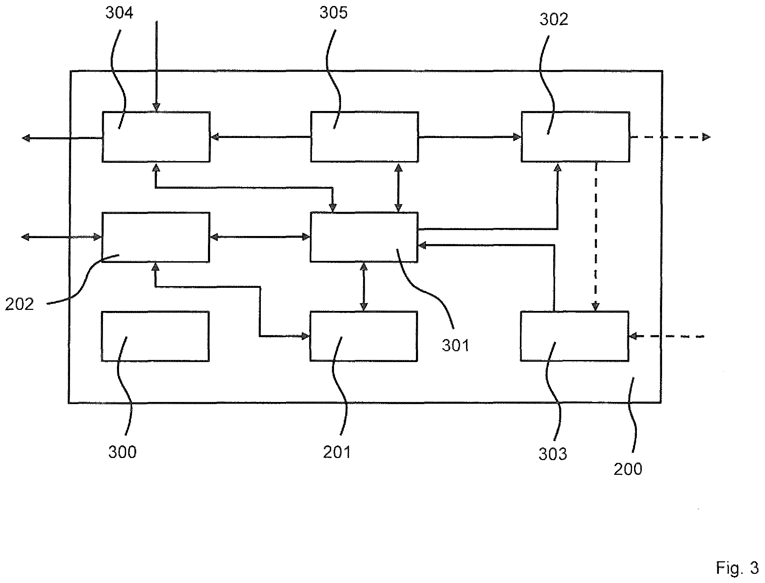

Fig. 1 shows a schematic representation of a medical system comprising a medical device 100, a communication device 101 and a safety device 102. The communication device 101 comprises a processing device 103, a memory device 104 and a transceiver 105. The safety device 102 comprises a control device 106, a trusted comparator 107 and a transceiver 108. The medical device 100 may comprise any elements necessary for the desired function of the medical device 100. In the embodiment shown, the medical device 100 is an insulin pump that is configured to be controlled by a medical device application running on the communication device 101. In alternative embodiments, the medical device 100 may be any medical device that may be controlled via the communication device 101. An exemplary embodiment of a medical device 100 is described below with reference to Fig. 3.

The processing device 103 of the communication device 101 is configured to establish a connection to the medical device 100 via the transceiver 105 of the communication device 101. The transceiver 105 of the communication device 101 is configured for a wireless data connection to the medical device 100. The processing device 103 of the communication device 101 is further configured to establish a connection to the safety device 102 via the transceiver 105 of the communication device 101 and the transceiver 108 of the safety device 102. In the embodiment shown, the transceiver 105 of the communication device 101 and the transceiver 108 of the safety device 102 are configured for a wireless data connection between the communication device 101 and the safety device 102.

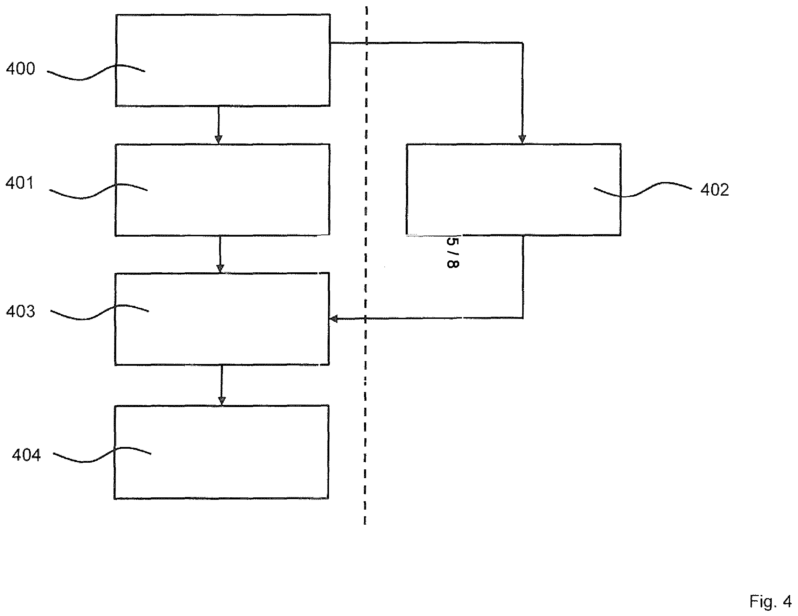

The processing device 103 of the communication device 101 is configured to execute a medical device application stored in the memory device 104. The control device 106 of the safety device 102 is configured to receive and transmit data via the transceiver 108 of the safety device 102. The control device 106 is further configured to control the trusted comparator 107. The trusted comparator 107 is configured to receive data from the control device 106, to perform steps of a method for controlling operation of the medical device 100, as laid out in more detail below with reference to Fig. 4 to 8, and to transmit data to the control device 106.

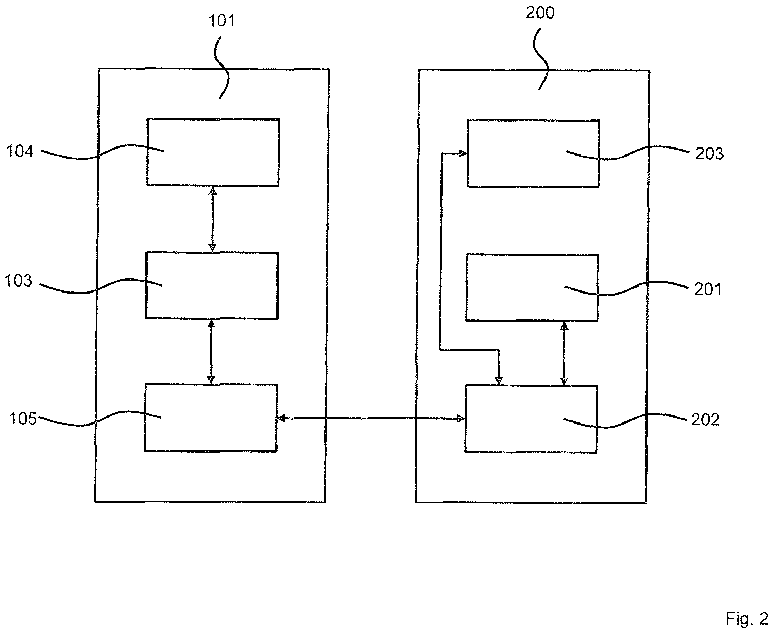

Fig. 2 shows a schematic representation of a different embodiment of a medical system. The medical system shown in Fig. 2 comprises a communication device 101 and a medical device 200. The communication device 101 corresponds to the communication device 101 shown in Fig. 1. The medical device 200 comprises a safety device 201 and a transceiver 202. The safety device 201 is provided as an integrated device of the medical device 200 and is configured to establish a connection to the communication device 101 via the transceiver 202 of the medical device 200. The transceiver 202 of the medical device 200 is configured for a wireless data connection to the transceiver 105 of the communication device 101. The safety device 201 comprises a control device and a trusted comparator (not shown) corresponding to the control device 106 and the trusted comparator 107 of the safety device 102 show in Fig. 1.

The medical device 200 comprises at least one further component 203 necessary for the desired function of the medical device 200. In the embodiment shown, the medical device 200 is an insulin pump that is configured to be controlled by a medical device application running on the communication device 101 and the at least one further component 203 is configured to provide the insulin pump functionality of the medical device 200. The processing device 103 of the communication device 101 is configured to establish a connection to the at least one further component 203 of the medical device 200 via the transceiver 105 of the communication device 101 and the transceiver 202 of the medical device 200 for controlling the medical device 200 by the medical device application.

Fig. 3 shows a schematic representation of an embodiment of a medical device 200 which, in the embodiment shown, is an insulin infusion pump. The medical device 200 comprises a power supply 300, configured to supply power to the medical device 200, and a control unit 301, configured to control function of the medical device 200. The control unit 301 is functionally connected to a transceiver 202 for data communication with devices external to the medical device 200, for a example a communication device 101. A safety device 201 is also functionally connected to the transceiver 202 for data communication with devices external to the medical device 200, in particular a communication device 101. The control device 301 controls the function of the safety device 201. A trusted comparator (not shown) provided in the safety device is functionally separate from other components of the medical device 200, including the control device 301.

The control device 301 further controls the insulin infusion pump provided by the medical device 200. An actuator unit 302 comprises at least one actuator, for example a pump device, for administering insulin to a user of the medical device 200. A sensor unit 303 comprises at least one sensor. For example, the sensor unit 303 may comprise a pressure sensor configured to measure a pressure in a reservoir containing insulin to be administered to a user of the medical device 200. The medical device 200 further comprises a user interface 304 configured for receiving user input, for example via a touchscreen and / or buttons of the user interface 304, and to transmit signals to a user of the medical device 200. Signals transmitted to a user of the medical device 200 may include visual, acoustic and / or tactile signals. For example, the user interface 304 may comprise a display, a loudspeaker and / or a vibration device for transmitting visual, acoustic and tactile signals, respectively.

A protection unit 305 enables and / or disables functioning of the actuator unit 302, thereby allowing or preventing administration of insulin to a user of the medical device 200. The protection unit 305 is configured to provide, via the user interface 304, user feedback to a user of the medical device 200 regarding the allowing and / or the preventing of administration of insulin. The protection unit 305 enables and / or disables functioning of the actuator unit 302 based on data and / or commands received from the control unit 301. Data and / or command provided to the protection unit 305 by the control unit 301 may be based, at least in part, on data and / or commands received in the control unit 301 from the safety device 201.

Fig. 4 shows steps of a method for controlling operation of a medical device 100 in a medical system in a flow diagram. In step 400 of the method, input data is provided in a medical device application running on a communication device 101, the medical device application adapted for remote operation control of the medical device 100. For example, the input data may be provided by user input in the communication device 101. Alternatively, input data may be received in the communication device 101 via data communication. As a further alternative, input data may be provided as a result of an operation, for example a calculation operation, in the communication device 101. The input data is encrypted in the communication device 101 and transmitted to a safety device 102.

Following, in step 401, the input data is processed in the communication device 101 in a first calculation process resulting in first calculation result data. In step 402, in the safety device 102, the input data is decrypted and processed in a second calculation process resulting in second calculation result data. The second calculation result data is encrypted and transmitted from the safety device 102 to the communication device 101. Steps 401 and 402 may be executed in any order or in parallel.

The second calculation result data is decrypted and compared to the first calculation result data in the communication device 101 in step 403. In step 404, if the first and second calculation result data are found equal, remote control operation of the medical device 100 by the communication device 101 is allowed. On the other hand, if the first and second calculation result data are found not equal, remote control operation of the medical device 100 by the communication device 101 is prevented in step 404.

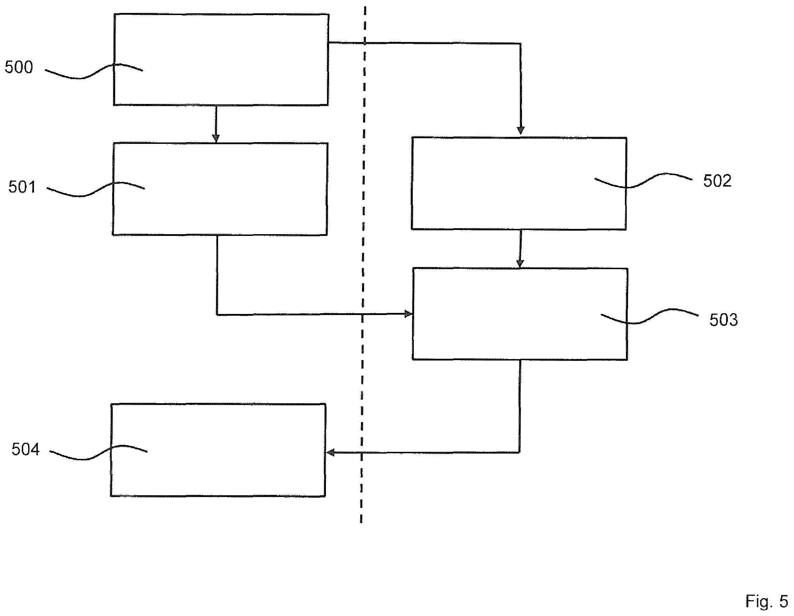

Fig. 5 shows steps of a different embodiment of a method for controlling operation of a medical device 100 in a medical system further comprising a communication device 101 and a safety device 102. Step 500 corresponds to step 400 described with reference to Fig. 4 above. In step 501, the input data is processed in the communication device 101 in a first calculation process resulting in first calculation result data. The first calculation result data is encrypted and transmitted from the communication device 101 to the safety device 102. In step 502, in the safety device 102, the input data is decrypted and processed in a second calculation process resulting in second calculation result data. Steps 501 and 502 may be executed in any order or in parallel.

The first calculation result data is decrypted and compared to the second calculation result data in the safety device 102 in step 503 resulting in compare result data indicative of the result of comparing the first and second calculation result data. The compare result data is encrypted and transmitted from the safety device 102 to the communication device 101. In step 504, the compare result data is decrypted in the communication device 101 and, if the first and second calculation result data are found equal, remote control operation of the medical device 100 by the communication device 101 is allowed. On the other hand, if the first and second calculation result data are found not equal, remote control operation of the medical device 100 by the communication device 101 is prevented in step 504.

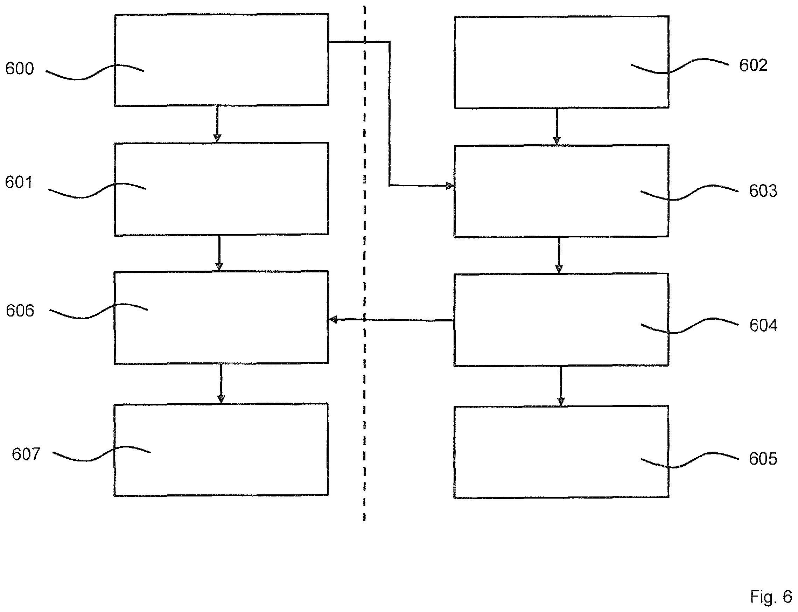

Fig. 6 shows steps of a different embodiment of a method for controlling operation of a medical device 100 in a medical system further comprising a communication device 101 and a safety device 102. In step 600, input data is provided in a medical device application running on a communication device 101. Step 600 corresponds to steps 400 and 500 described above with reference to Fig. 4 and 5, respectively. In step 601, corresponding to step 401 described with reference to Fig. 4 above, the input data is processed in the communication device 101 in a first calculation process resulting in first calculation result data.

In step 602, input reference data is provided in the safety device 102. The input reference data is indicative of valid input data. In step 603, in the safety device 102, the input data is decrypted and compared to the input reference data. In step 604, if the input data is found to be valid in step 603, the input data is processed in a second calculation process resulting in second calculation result data. The second calculation result data is encrypted and transmitted from the safety device 102 to the communication device 101. If in step 603 the input data is found to be not valid, input invalidity data is provided and encrypted in step 604 and transmitted from the safety device 102 to the communication device 101. In step 605, the second calculation result data or the input invalidity data as well as the input data are stored in a memory of the safety device 102. Additionally, the input reference data and / or any other data relevant to the processes described with reference to Fig. 6 may be stored in the safety device 102.

Steps 600 and 602 may be executed in any order or in parallel. Further, Step 601 may be executed before or after each of steps 603 and 604 or in parallel, at least partially.

In step 606, if encrypted input invalidity data is received in the communication device 101, the input invalidity data is decrypted. Following, remote control operation of the medical device 100 by the communication device 101 is prevented in step 607.

If encrypted second calculation result data is received in the communication device 101, the encrypted second calculation result data is decrypted and compared to the first calculation result data in the communication device 101 in step 606. Following, in step 607, if the first and second calculation result data are found equal, remote control operation of the medical device 100 by the communication device 101 is allowed. On the other hand, if the first and second calculation result data are found not equal, remote control operation of the medical device 100 by the communication device 101 is prevented in step 607.

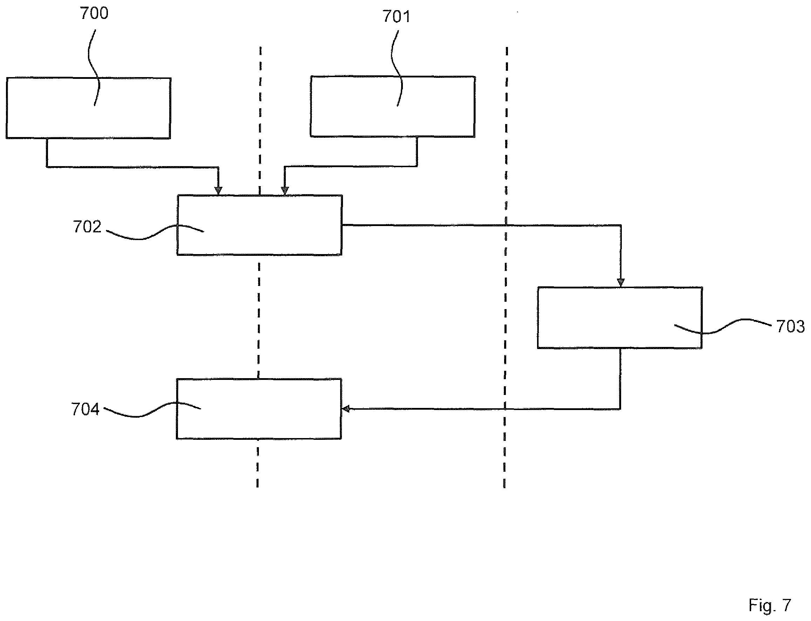

Fig. 7 shows steps of a further embodiment of a method for controlling operation of a medical device 100 in a medical system further comprising a communication device 101 and a safety device 102. In step 700, input data is provided in a first channel of a medical device application running on a communication device 101, the medical device application adapted for remote operation control of the medical device 100. The input data is processed in the first channel in a first calculation process resulting in first calculation result data. In step 701 the input data is provided in a second channel of the medical device application, the second channel being separate from the first channel. For example, the first channel and the second channel may employ different hardware of the communication device 101 and use different methodology to perform the same calculations in the medical device application. The input data is processed in the second channel in a second calculation process resulting in second calculation result data in step 701. Steps 700 and 701 may be performed in any order or, at least partially, in parallel.

In step 702, the first and second calculation result data are compared resulting in compare result data indicative of the result of comparing the first and second calculation result data. The first calculation result data, the second calculation result data and the compare result data are encrypted and transmitted to the safety device 102. Additionally, the input data may be encrypted and transmitted to the safety device 102.

In step 703, in the safety device 102, the first calculation result data, the second calculation result data and the compare result data are decrypted and the first and second calculation result data are compared to confirm the compare result data. Additionally, the input data may be decrypted and further confirmation functions may be executed. For example, third calculation result data may be generated by processing the input data in the safety device 102 in a third calculation process and the third calculation result data may be compared to the first and / or the second calculation result data to confirm the compare result data. Verdict data indicative of confirmation of the compare result data or failure to confirm the compare result data is provided in the safety device 102. The verdict data, the first calculation result data, the second calculation result data and the compare result data are encrypted and transmitted to the communication device 101.

The verdict data, the first calculation result data, the second calculation result data and the compare result data are decrypted in the communication device 101 in step 704. If the first and second calculation result data are found equal and the compare result data is confirmed according to the verdict data, remote control operation of the medical device 100 by the communication device 101 is allowed. If the first and second calculation result data are found not equal and / or the compare result data is not confirmed according to the verdict data, remote control operation of the medical device 100 by the communication device 101 is prevented in step 704.

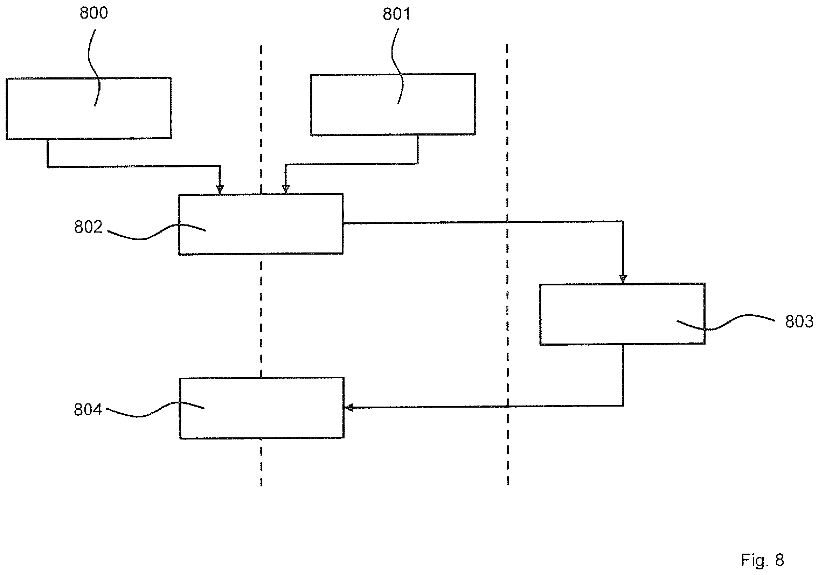

Fig. 8 illustrates a self test procedure in a method for controlling operation of a medical device 100 in a medical system further comprising a communication device 101 and a safety device 102. In step 800, first random data is created in a first channel of a medical device application running on a communication device 101, the medical device application adapted for remote operation control of the medical device 100. In step 801, second random data is created in a second channel of the medical device application, the second channel being separate from the first channel. Steps 800 and 801 may be performed in any order or, at least partially, in parallel.

In step 802, the first and second random data are compared resulting in compare result data indicative of the result of comparing the first and second random data. The first random data, the second random data and the compare result data are encrypted and transmitted to the safety device 102.

In step 803, in the safety device 102, the first random data, the second random data and the compare result data are decrypted and the first and second random data are compared to confirm the compare result data. Verdict data indicative of confirmation of the compare result data or failure to confirm the compare result data is provided in the safety device 102. The verdict data, the first random data, the second random data and the compare result data are encrypted and transmitted to the communication device 101.

The verdict data, the first random data, the second random data and the compare result data are decrypted in the communication device 101 in step 804. If the compare result data is confirmed according to the verdict data, remote control operation of the medical device 100 by the communication device 101 is allowed. If the compare result data is not confirmed according to the verdict data, remote control operation of the medical device 100 by the communication device 101 is prevented in step 804.

Embodiments of the methods described above with reference to Fig. 4 to 8 may apply accordingly to a safety device 201 provided as an integrated device of a medical device 200.

## Claims

1. A method for controlling operation of a medical device in a medical system, the medical system having
- a medical device;
- a communication device provided with a medical device application running on the communication device and adapted for remote operation control of the medical device; and
- a safety device adapted for data communication with the communication device;
the method, in the medical system, comprising:
- providing input data for a calculation process to the communication device and the safety device;
- receiving a software application at the communication device and the safety device for implementing the calculation process;
- processing the input data by the calculation process in a first calculation process performed by the software application on the communication device, thereby, providing first calculation result data;
- processing the input data by the calculation process in a second calculation process performed by the software application on the safety device and executed separately of the first calculation process, thereby, providing second calculation result data;
- comparing the first and second calculation result data;
- if the first and second calculation result data are found equal, allowing remote operation control of the medical device by the medical device application running on the communication device; and
- if the first and second calculation result data are found not equal, preventing the medical device application running on the communication device from remote operation control of the medical device.

2. Method according to claim 1, further comprising:
- providing the input data for the calculation process in both the communication device and the safety device; and
- processing the input data by the calculation process in the second calculation process in the safety device.

3. Method according to claim 2, further comprising
- receiving the input data in a first device of the communication device and the safety device;
- encrypting the input data in the first device;
- transmitting the encrypted input data from the first device to a second device of the communication device and the safety device; and
- decrypting the encrypted input data in the second device.

4. Method according to claim 1, further comprising:
- providing the input data for the calculation process in the communication device; and
- processing the input data by the calculation process in the second calculation process in the communication device.

5. Method according to any claim of the preceding claims, further comprising
- providing the first and second calculation result data in the safety device; and
- comparing the first and second calculation result data in the safety device.

6. Method according to claim 5, further comprising
- providing compare result data in the safety device, the compare result data indicative of the result of comparing the first and second calculation result data; and
- transmitting the compare result data from the safety device to the communication device.

7. Method according to claim 2 or 3 and claim 5 or 6, further comprising
- encrypting the first result data in the communication device;
- transmitting the encrypted first result data from the communication device to the safety device; and
- decrypting the encrypted first result data in the safety device.

8. Method according to claims 4 and 6, further comprising
- encrypting the first and second result data in the communication device;
- transmitting the encrypted first and second result data from the communication device to the safety device; and
- decrypting the encrypted first and second result data in the safety device.

9. Method according to any claim of the preceding claims, further comprising comparing the first and second calculation result data in the communication device.

10. Method according to claims 2 and 9, further comprising
- encrypting the second result data in the safety device;
- transmitting the encrypted second result data from the safety device to the communication device; and
- decrypting the encrypted second result data in the communication device.

11. Method according to any claim of the preceding claims, further comprising selecting the safety device from the following group: memory card such as SD card; NFC beacon; remote server device; further medical device different from the medical device.

12. Method according to any claim of the preceding claims, further comprising providing the calculation process and the comparing of the first and second calculation result data as safety elements of a single fault safety architecture.

13. Method according to any claim of the preceding claims, further comprising storing, in a memory device provided in at least one of the communication device and the safety device, at least one of the following: the input data; the encrypted input data; the first result data; the encrypted first result data; the second result data; and the encrypted second result data.

14. A medical system, comprising
- a medical device;
- a communication device provided with a medical device application running on the communication device and adapted for remote operation control of the medical device; and
- a safety device adapted for data communication with the communication device;
wherein the system is adapted to:
- provide input data for a calculation process to the communication device and the safety device;
- receiving a software application at the communication device and the safety device for implementing the calculation process;
- process the input data by the calculation process in a first calculation process performed by the software application on the communication device, thereby, providing first calculation result data;
- process the input data by the calculation process in a second calculation process performed by the software application on the safety device and executed separately of the first calculation process, thereby, providing second calculation result data;
- compare the first and second calculation result data;
- if the first and second calculation result data are found equal, allow remote operation control of the medical device by the medical device application running on the communication device; and
- if the first and second calculation result data are found not equal, prevent the medical device application running on the communication device from remote operation control of the medical device.

## Patentansprüche

1. Verfahren zum Steuern des Betriebs einer medizinischen Vorrichtung in einem medizinischen System, wobei das medizinische System Folgendes aufweist:
- eine medizinische Vorrichtung;
- eine Kommunikationsvorrichtung, die mit einer medizinischen Vorrichtungsanwendung versehen ist, die auf der Kommunikationsvorrichtung läuft und für die Fernbetriebssteuerung der medizinischen Vorrichtung ausgelegt ist; und
- eine Sicherheitsvorrichtung, die für die Datenkommunikation mit der Kommunikationsvorrichtung ausgelegt ist;
wobei das Verfahren in dem medizinischen System Folgendes umfasst:
- Bereitstellen von Eingabedaten für einen Berechnungsprozess an die Kommunikationsvorrichtung und die Sicherheitsvorrichtung;
- Empfangen einer Softwareanwendung an der Kommunikationsvorrichtung und der Sicherheitsvorrichtung zum Implementieren des Berechnungsprozesses;
- Verarbeiten der Eingabedaten durch den Berechnungsprozess in einem ersten Berechnungsprozess, der durch die Softwareanwendung auf der Kommunikationsvorrichtung durchgeführt wird, wodurch erste Berechnungsergebnisdaten bereitgestellt werden;
- Verarbeiten der Eingabedaten durch den Berechnungsprozess in einem zweiten Berechnungsprozess, der durch die Softwareanwendung auf der Sicherheitsvorrichtung durchgeführt wird und getrennt von dem ersten Berechnungsprozess ausgeführt wird, wodurch zweite Berechnungsergebnisdaten bereitgestellt werden;
- Vergleichen der ersten und zweiten Berechnungsergebnisdaten;
- wenn die ersten und zweiten Berechnungsergebnisdaten als gleich befunden werden, Zulassen der Fernbetriebssteuerung der medizinischen Vorrichtung durch die medizinische Vorrichtungsanwendung, die auf der Kommunikationsvorrichtung läuft; und
- wenn die ersten und zweiten Berechnungsergebnisdaten als nicht gleich befunden werden, Verhindern der Fernbetriebssteuerung der medizinischen Vorrichtung durch die medizinische Vorrichtungsanwendung, die auf der Kommunikationsvorrichtung läuft.

2. Verfahren nach Anspruch 1, das ferner Folgendes umfasst:
- Bereitstellen der Eingabedaten für den Berechnungsprozess sowohl in der Kommunikationsvorrichtung als auch in der Sicherheitsvorrichtung; und
- Verarbeiten der Eingabedaten durch den Berechnungsprozess in dem zweiten Berechnungsprozess in der Sicherheitsvorrichtung.

3. Verfahren nach Anspruch 2, das ferner Folgendes umfasst:
- Empfangen der Eingabedaten in einer ersten Vorrichtung der Kommunikationsvorrichtung und der Sicherheitsvorrichtung;
- Verschlüsseln der Eingabedaten in der ersten Vorrichtung;
- Übertragen der verschlüsselten Eingabedaten von der ersten Vorrichtung an eine zweite Vorrichtung der Kommunikationsvorrichtung und der Sicherheitsvorrichtung; und
- Entschlüsseln der verschlüsselten Eingabedaten in der zweiten Vorrichtung.

4. Verfahren nach Anspruch 1, das ferner Folgendes umfasst:
- Bereitstellen der Eingabedaten für den Berechnungsprozess in der Kommunikationsvorrichtung; und
- Verarbeiten der Eingabedaten durch den Berechnungsprozess in dem zweiten Berechnungsprozess in der Kommunikationsvorrichtung.

5. Verfahren nach einem der vorhergehenden Ansprüche, das ferner Folgendes umfasst:
- Bereitstellen der ersten und zweiten Berechnungsergebnisdaten in der Sicherheitsvorrichtung; und
- Vergleichen der ersten und zweiten Berechnungsergebnisdaten in der Sicherheitsvorrichtung.

6. Verfahren nach Anspruch 5, das ferner Folgendes umfasst:
- Bereitstellen von Vergleichsergebnisdaten in der Sicherheitsvorrichtung, wobei die Vergleichsergebnisdaten das Ergebnis des Vergleichs der ersten und zweiten Berechnungsergebnisdaten angeben; und
- Übertragen der Vergleichsergebnisdaten von der Sicherheitsvorrichtung an die Kommunikationsvorrichtung.

7. Verfahren nach Anspruch 2 oder 3 und Anspruch 5 oder 6, das ferner Folgendes umfasst:
- Verschlüsseln der ersten Ergebnisdaten in der Kommunikationsvorrichtung;
- Übertragen der verschlüsselten ersten Ergebnisdaten von der Kommunikationsvorrichtung an die Sicherheitsvorrichtung; und
- Entschlüsseln der verschlüsselten ersten Ergebnisdaten in der Sicherheitsvorrichtung.

8. Verfahren nach Anspruch 4 und 6, das ferner Folgendes umfasst:
- Verschlüsseln der ersten und zweiten Ergebnisdaten in der Kommunikationsvorrichtung;
- Übertragen der verschlüsselten ersten und zweiten Ergebnisdaten von der Kommunikationsvorrichtung an die Sicherheitsvorrichtung; und
- Entschlüsseln der verschlüsselten ersten und zweiten Ergebnisdaten in der Sicherheitsvorrichtung.

9. Verfahren nach einem der vorhergehenden Ansprüche, das ferner das Vergleichen der ersten und zweiten Berechnungsergebnisdaten in der Kommunikationsvorrichtung umfasst.

10. Verfahren nach Anspruch 2 und 9, das ferner Folgendes umfasst:
- Verschlüsseln der zweiten Ergebnisdaten in der Sicherheitsvorrichtung;
- Übertragen der verschlüsselten zweiten Ergebnisdaten von der Sicherheitsvorrichtung an die Kommunikationsvorrichtung; und
- Entschlüsseln der verschlüsselten zweiten Ergebnisdaten in der Kommunikationsvorrichtung.

11. Verfahren nach einem der vorhergehenden Ansprüche, das ferner das Auswählen der Sicherheitsvorrichtung aus der folgenden Gruppe umfasst: Speicherkarte wie etwa SD-Karte; NFC-Bake; entfernte Servervorrichtung; weitere medizinische Vorrichtung, die sich von der medizinischen Vorrichtung unterscheidet.

12. Verfahren nach einem der vorhergehenden Ansprüche, das ferner das Bereitstellen des Berechnungsprozesses und das Vergleichen der ersten und zweiten Berechnungsergebnisdaten als Sicherheitselemente einer Einzelfehler-Sicherheitsarchitektur umfasst.

13. Verfahren nach einem der vorhergehenden Ansprüche, das ferner das Speichern mindestens der Eingabedaten und/oder der verschlüsselten Eingabedaten und/oder der ersten Ergebnisdaten und/oder der verschlüsselten ersten Ergebnisdaten und/oder der zweiten Ergebnisdaten und/oder der verschlüsselten zweiten Ergebnisdaten in einer Speichervorrichtung, die in mindestens der Kommunikationsvorrichtung und/oder der Sicherheitsvorrichtung bereitgestellt ist, umfasst.

14. Medizinisches System, das Folgendes umfasst:
- eine medizinische Vorrichtung;
- eine Kommunikationsvorrichtung, die mit einer medizinischen Vorrichtungsanwendung versehen ist, die auf der Kommunikationsvorrichtung läuft und für die Fernbetriebssteuerung der medizinischen Vorrichtung ausgelegt ist; und
- eine Sicherheitsvorrichtung, die für die Datenkommunikation mit der Kommunikationsvorrichtung ausgelegt ist;
wobei das System für Folgendes ausgelegt ist:
- Bereitstellen von Eingabedaten für einen Berechnungsprozess an die Kommunikationsvorrichtung und die Sicherheitsvorrichtung;
- Empfangen einer Softwareanwendung an der Kommunikationsvorrichtung und der Sicherheitsvorrichtung zum Implementieren des Berechnungsprozesses;
- Verarbeiten der Eingabedaten durch den Berechnungsprozess in einem ersten Berechnungsprozess, der durch die Softwareanwendung auf der Kommunikationsvorrichtung durchgeführt wird, wodurch erste Berechnungsergebnisdaten bereitgestellt werden;
- Verarbeiten der Eingabedaten durch den Berechnungsprozess in einem zweiten Berechnungsprozess, der durch die Softwareanwendung auf der Sicherheitsvorrichtung durchgeführt wird und getrennt von dem ersten Berechnungsprozess ausgeführt wird, wodurch zweite Berechnungsergebnisdaten bereitgestellt werden;
- Vergleichen der ersten und zweiten Berechnungsergebnisdaten;
- wenn die ersten und zweiten Berechnungsergebnisdaten als gleich befunden werden, Zulassen der Fernbetriebssteuerung der medizinischen Vorrichtung durch die medizinische Vorrichtungsanwendung, die auf der Kommunikationsvorrichtung läuft; und
- wenn die ersten und zweiten Berechnungsergebnisdaten als nicht gleich befunden werden, Verhindern der Fernbetriebssteuerung der medizinischen Vorrichtung durch die medizinische Vorrichtungsanwendung, die auf der Kommunikationsvorrichtung läuft.

## Revendications

1. Procédé pour commander le fonctionnement d'un dispositif médical dans un système médical, le système médical ayant
- un dispositif médical ;
- un dispositif de communication pourvu d'une application de dispositif médical s'exécutant sur le dispositif de communication et adapté pour une commande de fonctionnement à distance du dispositif médical ; et
- un dispositif de sécurité adapté pour une communication de données avec le dispositif de communication ;
le procédé, dans le système médical, comprenant :
- la fourniture de données d'entrée pour un processus de calcul au dispositif de communication et au dispositif de sécurité ;
- la réception d'une application logicielle au niveau du dispositif de communication et du dispositif de sécurité pour mettre en œuvre le processus de calcul ;
- le traitement des données d'entrée par le processus de calcul dans un premier processus de calcul réalisé par l'application logicielle sur le dispositif de communication, fournissant ainsi des premières données de résultat de calcul ;
- le traitement des données d'entrée par le processus de calcul dans un deuxième processus de calcul réalisé par l'application logicielle sur le dispositif de sécurité et exécuté séparément du premier processus de calcul, fournissant ainsi des deuxièmes données de résultat de calcul ;
- la comparaison des premières et deuxièmes données de résultat de calcul ;
- si les premières et deuxièmes données de résultat de calcul sont déterminées comme étant égales, l'autorisation de la commande de fonctionnement à distance du dispositif médical par l'application de dispositif médical s'exécutant sur le dispositif de communication ; et
- si les premières et deuxièmes données de résultat de calcul sont déterminées comme n'étant pas égales, l'empêchement de l'application de dispositif médical s'exécutant sur le dispositif de communication de la commande de fonctionnement à distance du dispositif médical.

2. Procédé selon la revendication 1, comprenant en outre :
- la fourniture de données d'entrée pour le processus de calcul à la fois dans le dispositif de communication et dans le dispositif de sécurité ; et
- le traitement des données d'entrée par le processus de calcul dans le deuxième processus de calcul dans le dispositif de sécurité.

3. Procédé selon la revendication 2, comprenant en outre
- la réception des données d'entrée dans un premier dispositif parmi le dispositif de communication et le dispositif de sécurité ;
- le chiffrement des données d'entrée dans le premier dispositif ;
- la transmission des données d'entrée chiffrées du premier dispositif à un second dispositif parmi le dispositif de communication et le dispositif de sécurité ; et
- le déchiffrement des données d'entrée chiffrées dans le second dispositif.

4. Procédé selon la revendication 1, comprenant en outre :
- la fourniture de données d'entrée pour le processus de calcul dans le dispositif de communication ; et
- le traitement des données d'entrée par le processus de calcul dans le deuxième processus de calcul dans le dispositif de communication.

5. Procédé selon l'une quelconque des revendications précédentes, comprenant en outre
- la fourniture des premières et deuxièmes données de résultat de calcul dans le dispositif de sécurité ; et
- la comparaison des premières et deuxièmes données de résultat de calcul dans le dispositif de sécurité.

6. Procédé selon la revendication 5, comprenant en outre
- la fourniture de données de résultat de comparaison dans le dispositif de sécurité, les données de résultat de comparaison étant indicatives du résultat de la comparaison des premières et deuxièmes données de résultat de calcul ; et
- la transmission des données de résultat de comparaison du dispositif de sécurité au dispositif de communication.

7. Procédé selon la revendication 2 ou 3 et la revendication 5 ou 6, comprenant en outre
- le chiffrement des premières données de résultat dans le dispositif de communication ;
- la transmission des premières données de résultat chiffrées du dispositif de communication au dispositif de sécurité ; et
- le déchiffrement des premières données de résultat chiffrées dans le dispositif de sécurité.

8. Procédé selon les revendications 4 et 6, comprenant en outre
- le chiffrement des premières et deuxièmes données de résultat dans le dispositif de communication ;
- la transmission des premières et deuxièmes données de résultat chiffrées du dispositif de communication au dispositif de sécurité ; et
- le déchiffrement des premières et deuxièmes données de résultat chiffrées dans le dispositif de sécurité.

9. Procédé selon l'une quelconque des revendications précédentes, comprenant en outre la comparaison des premières et deuxièmes données de résultat de calcul dans le dispositif de communication.

10. Procédé selon les revendications 2 et 9, comprenant en outre
- le chiffrement des deuxièmes données de résultat dans le dispositif de sécurité ;
- la transmission des deuxièmes données de résultat chiffrées du dispositif de sécurité au dispositif de communication ; et
- le déchiffrement des deuxièmes données de résultat chiffrées dans le dispositif de communication.

11. Procédé selon l'une quelconque des revendications précédentes, comprenant en outre la sélection du dispositif de sécurité dans le groupe suivant : une carte mémoire telle qu'une carte SD ; une balise NFC ; un dispositif serveur distant ; un dispositif médical supplémentaire différent du dispositif médical.

12. Procédé selon l'une quelconque des revendications précédentes, comprenant en outre la fourniture du processus de calcul et la comparaison des premières et deuxièmes données de résultat de calcul en tant qu'éléments de sécurité d'une architecture de sécurité à défaut unique.

13. Procédé selon l'une quelconque des revendications précédentes, comprenant en outre le stockage, dans un dispositif de mémoire fourni dans au moins un parmi le dispositif de communication et le dispositif de sécurité, d'au moins une des suivantes : les données d'entrée ; les données d'entrée chiffrées ; les premières données de résultat ; les premières données de résultat chiffrées ; les deuxièmes données de résultat ; et les deuxièmes données de résultat chiffrées.

14. Système médical, comprenant
- un dispositif médical ;
- un dispositif de communication pourvu d'une application de dispositif médical s'exécutant sur le dispositif de communication et adapté pour une commande de fonctionnement à distance du dispositif médical ; et
- un dispositif de sécurité adapté pour une communication de données avec le dispositif de communication ;
dans lequel le système est adapté pour :
- fournir des données d'entrée pour un processus de calcul au dispositif de communication et au dispositif de sécurité ;
- recevoir une application logicielle au niveau du dispositif de communication et du dispositif de sécurité pour mettre en œuvre le processus de calcul ;
- traiter les données d'entrée par le processus de calcul dans un premier processus de calcul réalisé par l'application logicielle sur le dispositif de communication, fournissant ainsi des premières données de résultat de calcul ;
- traiter les données d'entrée par le processus de calcul dans un deuxième processus de calcul réalisé par l'application logicielle sur le dispositif de sécurité et exécuté séparément du premier processus de calcul, fournissant ainsi des deuxièmes données de résultat de calcul ;
- comparer les premières et deuxièmes données de résultat de calcul ;
- si les premières et deuxièmes données de résultat de calcul sont déterminées comme étant égales, autoriser la commande de fonctionnement à distance du dispositif médical par l'application de dispositif médical s'exécutant sur le dispositif de communication ; et
- si les premières et deuxièmes données de résultat de calcul sont déterminées comme n'étant pas égales, empêcher l'application de dispositif médical s'exécutant sur le dispositif de communication de la commande de fonctionnement à distance du dispositif médical.
